(19) Europäisches Patentamt
European Patent Office
Office européen des brevets

(11) **EP 4 702 982 A1**

(12) **EUROPEAN PATENT APPLICATION**
published in accordance with Art. 153(4) EPC

(43) Date of publication:
04.03.2026 Bulletin 2026/10

(21) Application number: 24797066.8

(22) Date of filing: 24.04.2024

(51) International Patent Classification (IPC):
*A61K 36/15* (2006.01)    *A23L 33/105* (2016.01)
*A61K 8/9767* (2017.01)    *A61P 17/14* (2006.01)
*A61P 25/24* (2006.01)    *A61Q 7/00* (2006.01)

(52) Cooperative Patent Classification (CPC):
A23L 33/105; A61K 8/9767; A61K 36/15;
A61P 17/14; A61P 25/24; A61Q 7/00

(86) International application number:
PCT/JP2024/016081

(87) International publication number:
WO 2024/225320 (31.10.2024 Gazette 2024/44)

(84) Designated Contracting States:
AL AT BE BG CH CY CZ DE DK EE ES FI FR GB
GR HR HU IE IS IT LI LT LU LV MC ME MK MT NL
NO PL PT RO RS SE SI SK SM TR
Designated Extension States:
BA
Designated Validation States:
GE KH MA MD TN

(30) Priority: 28.04.2023 JP 2023075024

(71) Applicants:
• Med R&D Co. Ltd
Tsukuba-shi, Ibaraki 305-8572 (JP)
• Arakawa Chemical Industries, Ltd.
Osaka-shi
Osaka 541-0046 (JP)

(72) Inventors:
• ISODA Hiroko
Tsukuba-shi, Ibaraki 305-8577 (JP)
• OGAWA IWAHASHI Hisako
Tsukuba-shi, Ibaraki 300-2611 (JP)
• FUJIOKA Daisuke
Tsukuba-shi, Ibaraki 300-2611 (JP)
• TAKAHASHI Koji
Tsukuba-shi, Ibaraki 300-2611 (JP)

(74) Representative: Hautier IP - MC/EP
17, avenue Albert II
C/O The Office & Co - L'ALBU
98000 Monaco (MC)

(54) **VITALITY IMPROVING AGENT, COMPOSITION FOR IMPROVING VITALITY, AND FOOD COMPOSITION FOR IMPROVING VITALITY**

(57) A vitality improving agent contains a pine needle extract as an active ingredient; a composition for improving vitality contains the vitality improving agent and a pharmaceutically acceptable carrier; and a food composition for improving vitality contains the vitality improving agent.

FIG. 6

EP 4 702 982 A1

**Description**

TECHNICAL FIELD

The present invention relates to a vitality improving agent, a composition for improving vitality, and a food composition for improving vitality. Priority is claimed on Japanese Patent Application No. 2023-075024, filed April 28, 2023, the content of which is incorporated herein by reference.

BACKGROUND ART

Various kinds of vitality decrease with aging. For example, along with aging, hair loss on the head, a decrease in neural function regulatory function, and a decrease in cognitive function occur. Furthermore, it is said that depressive symptoms also occur with aging.

Hair loss on the head is a symptom caused by various causes such as aging. Although hair loss is not a dangerous symptom per se, when the importance of hair loss on the head to the appearance is considered, the hair loss can have a significant impact on the quality of life of an individual. For this reason, various treatment methods for hair loss have been studied.

As a method for treating alopecia that can be currently utilized, methods using synthetic chemical components are mainly known. For example, "minoxidil" and "finasteride" have been approved by the U.S. Food and Drug Administration (FDA) as therapeutic agents for alopecia (see, for example, Patent Documents 1 and 2).

Furthermore, the present society is also referred to as a stressful society, and as a result of being forced to live a stressful life on a daily basis, the number of patients with mental illnesses such as depression is increasing, which has become a major social problem. The impact of these mental illnesses on society, such as the increase in medical expenses as well as economic losses and social losses due to suicide, is immeasurable.

Currently, various antidepressants such as bupropion, milnacipran, and imipramine have been developed and prescribed to patients with mental illnesses such as depression (see, for example, Patent Document 3).

Citation List

Patent Documents

Patent Document 1: United States Patent No. 4139619
Patent Document 2: United States Patent Application, Publication No. 2002/0042425
Patent Document 3: United States Patent Application, Publication No. 2004/121010

SUMMARY OF INVENTION

Technical Problem

However, the administration of an existing therapeutic agent for alopecia, such as minoxidil, may cause many serious side effects (impotence, dizziness, unintended hair growth, weakness, headache, skin rashes, and the like). Therefore, there is a demand for hair growth agents derived from natural products, which are expected to have fewer side effects and be safe.

Furthermore, many of the existing antidepressants are artificially synthesized low-molecular-weight compounds, and side effects such as nausea, loss of appetite, drowsiness, and low blood pressure are problematic. Furthermore, there is a problem that excessive intake of these antidepressants may lead to severe toxicity in many cases. Therefore, there is a demand for antidepressants derived from natural products, which are expected to have fewer side effects and be safe.

In this way, there is a demand for a technology for safely ameliorating loss of vitality associated with aging or the like, as represented by hair loss and depression. Thus, an object of the present invention is to provide a vitality improving agent derived from a natural product.

Solution to Problem

The present invention includes the following aspects.

[1] A vitality improving agent containing a pine needle extract as an active ingredient.

[2] The vitality improving agent according to [1], in which the pine needle extract is an extract obtained using water or a mixed liquid of water and an alcohol as an extraction solvent.

[3] The vitality improving agent according to [1] or [2], in which the pine needle extract contains 500 to 9,500 ppm by mass of D-pinitol and 30 to 8,500 ppm by mass of shikimic acid.

[4] The vitality improving agent according to any one of [1] to [3], in which the pine needle extract contains substantially no resin acids.

[5] A composition for improving vitality, containing:

the vitality improving agent according to any one of [1] to [4]; and
a pharmaceutically acceptable carrier.

[6] A food composition for improving vitality, containing the vitality improving agent according to any one of [1] to [4].

It can also be said that the present invention includes the following aspects.

[P1] A hair growth agent containing a pine needle extract as an active ingredient.

[P2] The hair growth agent according to [P1], in which the pine needle extract is an extract obtained using an alcohol or a mixed liquid of water and an alcohol as an extraction solvent.

[P3] A composition for hair growth, containing:

the hair growth agent according to [P1] or [P2]; and
a pharmaceutically acceptable carrier.

[P4] An antidepressant containing a pine needle extract as an active ingredient.

[P5] The antidepressant according to [P4], in which the pine needle extract is an extract obtained using an alcohol or a mixed liquid of water and an alcohol as an extraction solvent.

[P6] A composition for treating depression, containing:

the antidepressant according to [P4] or [P5]; and
a pharmaceutically acceptable carrier.

Advantageous Effects of Invention

According to the present invention, a vitality improving agent derived from a natural product can be provided.

BRIEF DESCRIPTION OF DRAWINGS

[FIG. 1] FIG. 1 is a graph showing results of measuring cell viability in Experimental Example 4.

[FIG. 2] FIG. 2 is a graph showing the results of quantitative real-time PCR of CTNNB1 gene in Experimental Example 6.

[FIG. 3] FIG. 3 is a graph showing the results of quantitative real-time PCR of ALPL gene in Experimental Example 6.

[FIG. 4] FIG. 4 is a graph showing the results of quantitative real-time PCR of FGF1 gene in Experimental Example 6.

[FIG. 5] FIG. 5 is a graph showing the results of evaluating cell proliferation in Experimental Example 8.

[FIG. 6] FIG. 6 is a graph showing the results of a tail suspension test in Experimental Example 9.

[FIG. 7] FIG. 7 is a graph showing the results of Experimental Example 10.

[FIG. 8] FIG. 8 is a view showing the results of performing Gene Ontology (GO) analysis on a gene group whose expression has been altered in a high-dose pine needle extract-administered group in Experimental Example 11.

DESCRIPTION OF EMBODIMENTS

[Vitality improving agent]

In one embodiment, the present invention provides a vitality improving agent containing a pine needle extract as an active ingredient. As will be described later in the Examples, by administering the vitality improving agent according to the present embodiment, loss of vitality associated with aging in a human or a non-human animal can be ameliorated or treated. Examples of the loss of vitality associated with aging include hair loss and depression.

Examples of the non-human animal include pet animals and livestock. Examples of the pet animals include a dog, a cat, a rabbit, and a hamster. Examples of the livestock include a horse and a cow.

[Vitality improving agent (hair growth agent)]

The vitality improving agent of the present invention has hair growth effects in a human or a non-human animal. For the non-human animal, the same applies as described above. Therefore, in one embodiment, the present invention can be said to provide a hair growth agent containing a pine needle extract as an active ingredient. As will be described later in the Examples, the inventors have found that a pine needle extract has hair growth promoting effects. The hair growth agent of the present embodiment can be referred to as a therapeutic agent for alopecia, a hair restorer, or the like.

In the hair growth agent of the present embodiment, the phrase "containing as an active ingredient" means that the hair growth agent contains a pine needle extract in an amount sufficient to exhibit effects as a hair growth agent. Alternatively, the phrase means that the hair growth agent contains a pine needle extract as a main active component. Examples of the effects as a hair growth agent include promoting hair growth. Alternative examples of the effects include increasing the expression level of a hair growth-related gene in the hair papilla cells in the presence of the hair growth agent, as compared with the expression level of the hair growth-related gene in the absence of the hair growth agent. Examples of the hair growth-related gene include CTNNB1 gene (β-catenin), ALPL gene (alkaline phosphatase), and FGF1 gene (Fiblobrast growth factor 1). As will be described later in the Examples, the hair growth agent of the present embodiment can significantly increase the expression level of a hair growth-related gene in the hair papilla cells. Therefore, the hair growth agent of the present embodiment can also be said to be an expression promoter for CTNNB1 gene, ALPL gene, or FGF1 gene.

The pine needles used as a material for the pine needle extract may be leaves of a plant belonging to the genus Pinus of the family Pinaceae (pine). The type of pine is not particularly limited, and examples thereof include Pinus densiflora, P. densata, P. fragilissima, P. heldreichii, P. henryi, P. hwangshanensis, P. kesiya, P. luchuensis Mayr, P. massoniana, P. mugo, P. nigra, P. resinosa, P. sylvestris, P. tabuliformis, P. taiwanensis, P. thunbergii, P. tropicalis, P. yunnanensis, P. pinea, P. brutia, P. canariensis, P. halepensis, P. latteri, P. merkusii, P. pinaster Ait., P. roxburghii, P. leiophylla, P. lumholtzii, P. caribaea, P. cubensis, P. echinata, P. elliottii, P. glabra, P. occidentalis, P. palustris, P. pungens, P. rigida Mill., P. serotina, P. taeda, P. banksiana, P. clausa, P. contorta, P. virginiana, P. attenuata, P. foisyi, P. greggii, P. herrerae, P. jaliscana, P. lawsonii, P. muricata, P. oocarpa, P. patula, P. praetermissa, P. pringlei, P. radiata D. Don, P. tecunumanii, P. teocote, P. apulcensis, P. arizonica, P. cooperi, P. coulteri, P. devoniana, P. durangensis, P. engelmannii, P. hartwegii, P. jeffreyi, P. johndayensis, P. maximinoi, P. montezumae, P. ponderosa, P. pseudostrobus, P. sabiniana, P. torreyana, P. amamiana, P. armandii, P. ayacahuite, P. bhutanica, P. chiapensis, P. dabeshanensis, P. dalatensis, P. fenzeliana, P. flexilis, P. reflexa, P. lambertiana, P. morrisonicola, P. monticola, P. parviflora, P. peuce, P. pumila, P. strobiformis, P. strobus, P. wallichiana, P. wangii, P. albicaulis, P. cembra, P. koraiensis, P. sibirica, P. nelsonii, P. krempfii, P. bungeana, P. gerardiana, P. squamata, P. maximartinezii, P. pinceana, P. rzedowskii, P. cembroides, P. culminicola, P. discolor, P. edulis, P. johannis, P. monophylla, P. orizabensis, P. quadrifolia, P. remota, P. aristata, P. balfouriana, and Pinus longaeva.

Pine needles may be subjected to an extraction operation in a fresh state or may be subjected to an extraction operation after being dried. Furthermore, pine needles may also be subjected to an extraction operation after being crushed or ground.

Examples of the extraction solvent include water, an alcohol, and a mixture of water and an alcohol. The alcohol is not particularly limited, and examples thereof include lower alcohols having 1 to 4 carbon atoms, such as ethanol, methanol, propanol, butanol, and 1,3-butanediol. Regarding the alcohol, one kind thereof may be used alone, or two or more kinds thereof may be used in combination.

Water may be mixed with an alcohol to be used as an extraction solvent. In the hair growth agent of the present embodiment, it is preferable that the extraction solvent is water or a mixed liquid of water and an alcohol. The concentration of alcohol in the mixed liquid of water and the alcohol may be 10% to 98% by volume; may be, for example, 20% to 98% by volume; may be, for example, 40% to 98% by volume; may be, for example, 60% to 98% by volume; or may be, for example, about 70% by volume.

It is particularly preferable that the extraction solvent is water. As will be described later in Examples, when water is used as the extraction solvent, the pine needle extract tends to have low cytotoxicity.

As a method for extracting pine needles, a generally used method can be employed. For example, a method of immersing pine needles in the above-described extraction solvent and performing extraction may be mentioned. When the extraction solvent is a mixed liquid of water and an alcohol, the extraction temperature may be, for example, 1°C to 50°C, may be 10°C to 30°C, or may be room temperature of 15°C to 25°C. In this case, the extraction time may be several hours to several weeks, and may be, for example, 1 to 10 days; may be, for example, 3 to 10 days; may be, for example, 5 to 10 days; or may be, for example, about 7 days. Furthermore, the extraction solvent may be stirred during the extraction.

When the extraction solvent is water, the extraction temperature may be 60°C or higher, may be 70°C or higher, may be 80°C or higher, may be 90°C or higher, or may be 100°C. Furthermore, pressure may be applied during the extraction. When pressure is applied, the extraction temperature may exceed 100°C. For example, the extraction temperature may be 100°C or higher, may be 105°C or higher, or may be 110°C or higher. In this case, the extraction time may be 24 hours or less, may be 12 hours or less, may be 6 hours or less, may be 3 hours or less, may be 1 hour or less, or may be about 30 minutes. Furthermore, the extraction solvent may be stirred during the extraction. In the present specification, the term "about" means inclusive of ±10%. Therefore, a period of about 7 days may be 6.3 to 7.7 days. Furthermore, a period of about 30 minutes may be 27 to 33 minutes.

When the extraction solvent is water, the extraction temperature is preferably 60°C or higher. Furthermore, in this case, the extraction time is preferably 24 hours or less.

The obtained pine needle extract may be used as it is after removing solid matter by filtration or centrifugation as necessary, may be used as a soft extract after distilling off the solvent, or may be used as an extract dried product after drying by reduced pressure drying, freeze-drying, or the like.

It is preferable that the pine needle extract contains 500 ppm by mass or more of D-pinitol, more preferably contains 700 ppm by mass or more of D-pinitol, even more preferably contains 1,000 ppm by mass or more of D-pinitol, and particularly preferably contains 2,000 ppm by mass or more of D-pinitol, and it is preferable that the pine needle extract contains 9,500 ppm by mass or less of D-pinitol, and more preferably contains 5,000 ppm by mass or less of D-pinitol. Furthermore, it is preferable that the pine needle extract contains 30 ppm by mass or more of shikimic acid, more preferably contains 100 ppm by mass or more of shikimic acid, even more preferably contains 1,000 ppm by mass or more of shikimic acid, and particularly preferably contains 2,000 ppm by mass or more of shikimic acid, and it is preferable that the pine needle extract contains 8,500 ppm by mass or less of shikimic acid. In addition, it is preferable that the pine needle extract contains 500 to 9,500 ppm by mass of D-pinitol and 30 to 8,500 ppm by mass of shikimic acid, and more preferably contains 2,000 to 9,500 ppm by mass of D-pinitol and 2,000 to 8,500 ppm by mass of shikimic acid. A pine needle extract extracted under the conditions of containing these components at the above-described contents tends to have high expression enhancing effects for hair growth-related genes. The amounts of D-pinitol and shikimic acid in the present embodiment are values calculated relative to a case where the non-volatile content of the pine needle extract is set to 3% by mass, as will be described later in the Examples.

It is preferable that the pine needle extract contains substantially no resin acids. The resin acids are a main component of rosin, and mean a mixture containing various isomers of abietic acid, dehydroabietic acid, palustric acid, pimaric acid, and communic acid, and the like as main components. The resin acids also include resin acid derivatives such as hydrogenated resin acids. As will be described later in the Examples, a pine needle extract having a high content of resin acids tends to have high cytotoxicity.

Here, the phrase "contains substantially no resin acids" means that it is acceptable for the pine needle extract to contain resin acids to the extent that the cytotoxicity does not pose a problem. When the total amount of resin acids is about 200 ppm by mass or less, it can be said that the pine needle extract contains substantially no resin acids.

It is preferable that the vitality improving agent of the present embodiment is formulated as a composition for improving vitality, which contains a pharmaceutically acceptable carrier. The composition for improving vitality may be a pharmaceutical composition. When attention is paid to the hair growth effects of the vitality improving agent, the composition for improving vitality of the present embodiment can be referred to as a composition for hair growth or a composition for hair restoration. In one aspect, the composition for hair growth contains a pine needle extract and a pharmaceutically acceptable carrier.

The pharmaceutically acceptable carrier is not particularly limited, and examples thereof include an excipient, a binder, a disintegrant, a lubricating agent, an emulsifier, a thickener, a wetting agent, and a solvent for an injectable preparation. Furthermore, the composition for hair growth may further contain additives.

The additives are not particularly limited, and examples thereof include an antiseptic agent, a pH adjuster, a stabilizer, an ultraviolet absorber, an antioxidant, a colorant, and a flavor.

As the pharmaceutically acceptable carrier and the additives, for example, those general raw materials described in the 18th edition of the Japanese Pharmacopoeia and the like can be used.

Examples of the dosage form of the composition for hair growth include orally administrable dosage forms such as a tablet, a coated tablet, a pill, a powder, a granular preparation, a capsule, a liquid preparation, a suspension, and an emulsion; and parenterally administrable dosage forms such as an injectable preparation, a suppository, and a topical skin preparation.

Examples of the topical skin preparation include dosage forms such as a cream, a lotion, a skin toner, an emulsion, a foundation, a pack preparation, a foam preparation, a plaster, an ointment, a patch preparation, and an aerosol agent.

The composition for hair growth may be a therapeutic agent for alopecia, may be a cosmetic, or may be a food such as a supplement.

The content of the hair growth agent (pine needle extract) in the composition for hair growth is, for example, in the range of 0.01% to 50% by mass, 0.01% to 30% by mass, 0.01% to 10% by mass, 0.01% to 5% by mass, or 0.01% by mass, in terms of the solid content (dry mass) of the hair growth agent.

A method for administering the hair growth agent or the composition for hair growth is not particularly limited and may be appropriately determined according to the symptoms, body weight, age, sex, and the like of the administration target. For example, a tablet, a coated tablet, a pill, a powder, a granular preparation, a capsule, a liquid preparation, a suspension, or an emulsion is administered orally. Furthermore, the injectable preparation is intravenously administered singly or as a mixture with conventional replenishing fluids such as glucose and amino acids, and as necessary, the injectable preparation is administered intraarterially, intramuscularly, intradermally, subcutaneously, or intraperitoneally. A suppository is intrarectally administered. A topical skin preparation is applied, affixed, or sprayed onto an affected area.

The amount of administration of the hair growth agent or the composition for hair growth varies depending on the symptoms, body weight, age, sex, and the like of the administration target and cannot be determined in a general manner; however, in the case of oral administration, for example, 0.01 to 5,000 mg/kg of body weight per day of the active ingredient (pine needle extract) may be administered. Furthermore, in the case of an injectable preparation, for example, 0.01 to 500 mg per day of the active ingredient may be administered. Furthermore, in the case of a suppository, for example, 0.01 to 1,000 mg per day of the active ingredient may be administered. Furthermore, in the case of a topical skin preparation, for example, 0.01 to 500 mg per day of the active ingredient may be administered.

[Food composition for improving vitality (food composition for hair growth)]

In one embodiment, the present invention provides a food composition for hair growth, which contains a pine needle extract as an active ingredient. The food composition of the present embodiment contains the above-described hair growth agent. By taking in the food composition of the present embodiment, hair growth can be promoted in humans or non-human animals. Alternatively, by taking in the food composition of the present embodiment, the expression level of hair growth-related gene in hair papilla cells can be increased in a human or a non-human animal. For the non-human animal, the same applies as described above. The food composition of the present embodiment can also be referred to as a food composition for promoting hair growth, a food composition for hair restoration, or the like.

A preferred intake amount of the food composition of the present embodiment is the same as that of the above-mentioned hair growth agent or composition for hair growth, and examples thereof include an amount allowing intake of 0.01 to 5,000 mg/kg of body weight per day of the active ingredient (pine needle extract). The food composition may be taken in once a day or in divided doses of about 2 to 4 times a day.

The food composition of the present embodiment may be, for example, in the form of a supplement, may be in the form of a beverage, may be in the form of a solid, semi-solid, or gel-like food, or may be in the form of any pre-cooked food.

Examples of the shape of the supplement include a powder, a granular preparation, a tablet, and a capsule. Examples of the beverage include coffee, tea, black tea, sports drinks, fruit and vegetable drinks, and carbonated drinks.

The food composition of the present embodiment may be a food with functional claims. The "food with functional claims" means a food that has been reported to the Consumer Affairs Agency as claiming functionality displayed on the product package based on scientific grounds.

[Vitality improving agent (antidepressant)]

A vitality improving agent of the present invention has antidepressant effects in a human or a non-human animal. For the non-human animal, the same applies as described above. Therefore, in one embodiment, the present invention can be said to provide an antidepressant containing a pine needle extract as an active ingredient. As will be described later in the Examples, the inventors have found that a pine needle extract has a neuroprotective action. Since the antidepressant of the present embodiment is derived from a natural product, the antidepressant is considered to be safe with fewer side effects.

In the antidepressant of the present embodiment, the phrase "containing as an active ingredient" means that the antidepressant contains a pine needle extract in an amount sufficient to exhibit effects as an antidepressant. Alternatively, the phrase means that the antidepressant contains a pine needle extract as a main active component. Examples of the effects as an antidepressant include exhibiting a neuroprotective action.

The neuroprotective action can be referred to as an antidepressant action, an anti-stress action, or the like. That is, the antidepressant of the present embodiment can be referred to as a therapeutic agent for depression, a neuroprotective agent, an anti-stress agent, or the like.

In the antidepressant of the present embodiment, the pine needle extract is the same as described above. The pine needle extract is preferably an extract obtained using ethanol or a mixed liquid of water and ethanol as an extraction solvent, and more preferably an extract obtained using water as an extraction solvent.

It is preferable that the vitality improving agent of the present embodiment is formulated as a composition for improving vitality, which contains a pharmaceutically acceptable carrier. The composition for improving vitality may be a pharmaceutical composition. When attention is paid to the antidepressant effects of the vitality improving agent, the composition for improving vitality of the present embodiment can be said to be a composition for treating depression. In one aspect, the composition for treating depression contains a pine needle extract and a pharmaceutically acceptable carrier.

Examples of the pharmaceutically acceptable carrier include carriers similar to those described above for the composition for hair growth. Furthermore, the composition for treating depression may further contain additives, and the additives are also similar to those described above. As the pharmaceutically acceptable carrier and the additives, for example, those general raw materials described in the 18th edition of the Japanese Pharmacopoeia and the like can be used.

Examples of the dosage form of the composition for treating depression include orally administrable dosage forms such as a tablet, a coated tablet, a pill, a powder, a granular preparation, a capsule, a liquid preparation, a suspension, and an emulsion; and parenterally administrable dosage forms such as an injectable preparation, a suppository, and a topical skin preparation.

Examples of the topical skin preparation include dosage forms such as a cream, a lotion, a skin toner, an emulsion, a foundation, a pack preparation, a foam preparation, a plaster, an ointment, a patch preparation, and an aerosol agent.

The composition for treating depression may be a therapeutic agent for depression, may be a cosmetic, or may be a food such as a supplement.

The content of the antidepressant (pine needle extract) in the composition for treating depression is, for example, in the range of 0.01% to 50% by mass, 0.01% to 30% by mass, 0.01% to 10% by mass, 0.01% to 5% by mass, or 0.01% to 1% by mass, in terms of the solid content (dry mass) of the antidepressant.

A method for administering the antidepressant or the composition for treating depression is not particularly limited and may be appropriately determined according to the symptoms, body weight, age, sex, and the like of the administration target. For example, a tablet, a coated tablet, a pill, a powder, a granular preparation, a capsule, a liquid preparation, a suspension, or an emulsion is administered orally. Furthermore, the injectable preparation is intravenously administered singly or as a mixture with conventional replenishing fluids such as glucose and amino acids, and as necessary, the injectable preparation is administered intraarterially, intramuscularly, intradermally, subcutaneously, or intraperitoneally. A suppository is intrarectally administered. A topical skin preparation is applied, affixed, or sprayed onto an affected area.

The amount of administration of the antidepressant or the composition for treating depression varies depending on the symptoms, body weight, age, sex, and the like of the administration target and cannot be determined in a general manner; however, in the case of oral administration, for example, 0.01 to 5,000 mg/kg of body weight per day of the active ingredient (pine needle extract) may be administered. Furthermore, in the case of an injectable preparation, for example, 0.01 to 500 mg per day of the active ingredient may be administered. Furthermore, in the case of a suppository, for example, 0.01 to 1,000 mg per day of the active ingredient may be administered. Furthermore, in the case of a topical skin preparation, for example, 0.01 to 500 mg per day of the active ingredient may be administered.

[Food composition for improving vitality (food composition for ameliorating depression)]

In one embodiment, the present invention provides a food composition for ameliorating depression, which contains a pine needle extract as an active ingredient. The food composition of the present embodiment contains the above-described antidepressant. By taking in the food composition of the present embodiment, a neuroprotective action can be exhibited in a human or a non-human animal. The neuroprotective action can be referred to as an antidepressant action, an anti-stress action, or the like. That is, the food composition of the present embodiment can be referred to as a food composition for ameliorating depression, a food composition for neuroprotection, a food composition for anti-stress, or the like.

A preferred intake amount of the food composition of the present embodiment is the same as that of the above-mentioned hair growth agent or composition for hair growth, and examples thereof include an amount allowing intake of 0.01 to 5,000 mg/kg of body weight per day of the active ingredient (pine needle extract). The food composition may be taken in once a day or in divided doses of about 2 to 4 times a day.

The food composition of the present embodiment may be, for example, in the form of a supplement, may be in the form of a beverage, may be in the form of a solid, semi-solid, or gel-like food, or may be in the form of any pre-cooked food.

Examples of the shape of the supplement include a powder, a granular preparation, a tablet, and a capsule. Examples of the beverage include coffee, tea, black tea, sports drinks, fruit and vegetable drinks, and carbonated drinks.

The food composition of the present embodiment may be a food with functional claims. The "food with functional claims" means a food that has been reported to the Consumer Affairs Agency as claiming functionality displayed on the product package based on scientific grounds.

[Other embodiments]

In one embodiment, the present invention provides a method for treating loss of vitality associated with aging, the method including administering an effective amount of a pine needle extract to a patient in need of treatment.

In one embodiment, the present invention provides a pine needle extract for use in the treatment of loss of vitality associated with aging.

In one embodiment, the present invention provides the use of a pine needle extract for producing a therapeutic agent for loss of vitality associated with aging.

In one embodiment, the present invention provides a method for treating alopecia, the method including administering an effective amount of a pine needle extract to a patient in need of treatment.

In one embodiment, the present invention provides a pine needle extract for use in the treatment of alopecia.

In one embodiment, the present invention provides the use of a pine needle extract for producing a therapeutic agent for alopecia.

In one embodiment, the present invention provides a method for treating depression, the method including administering an effective amount of a pine needle extract to a patient in need of treatment.

In one embodiment, the present invention provides a pine needle extract for use in the treatment of depression.

In one embodiment, the present invention provides the use of a pine needle extract for producing a therapeutic agent for depression.

In each of these embodiments, the pine needle extract, the form of administration, the amount of administration, and the like are the same as described above. It is preferable that the pine needle extract is formulated as a pharmaceutical composition containing a pharmaceutically acceptable carrier. The pharmaceutically acceptable carrier is the same as described above.

Examples

Next, the present invention will be described in more detail by way of Examples; however, the present invention is not intended to be limited to the following Examples.

[Experimental Example 1]

(Preparation of pine needle extract of Production Example 1)

A dried product of pine needles derived from seedlings of Japanese red pine was ground with a mixer. Subsequently, 100 mg of the ground product of pine needles and 1 mL of a 70% by volume aqueous solution of ethanol were mixed, extraction was performed in a dark place at room temperature for 1 week, and a pine needle extract of Production Example 1 was obtained. During the extraction, the sample was stirred with a vortex every day. Subsequently, the mixture was centrifuged at 1,000 $\times$ g for 15 minutes to collect the supernatant. Subsequently, the supernatant was sterilized by passing through a filter having a pore size of 0.22 $\mu$m and stored at -20°C. The pine needle extract was used after directly diluting the pine needle extract in the culture medium of cells used in each experiment.

[Experimental Example 2]

(Preparation of pine needle extracts of Production Examples 2 to 7)

The pine needles shown in the following Table 1 were each extracted by the extraction methods shown in the following Table 1. Subsequently, the mixture was centrifuged at 1,000 $\times$ g for 15 minutes to collect the supernatant. Subsequently, the supernatant was sterilized by passing the supernatant through a filter having a pore size of 0.22 $\mu$m, and pine needle extracts of Production Examples 2 to 7 were obtained. Each of the pine needle extracts was stored at -20°C.

[Table 1]

| Production Example | Origin of pine needles | Extraction method |
|---|---|---|
| 2 | Japanese red pine, dried product of pine needles derived from seedlings | Extraction with water at 105°C for 30 minutes |
| 3 | Japanese red pine, dried product of pine needles derived from adult trees | Extraction with water at 105°C for 30 minutes |

(continued)

| Production Example | Origin of pine needles | Extraction method |
|---|---|---|
| 4 | Japanese red pine, dried product of pine needles derived from adult trees | Extraction with water at 105°C for 30 minutes |
| 5 | Japanese red pine, dried product of pine needles derived from seedlings | Extraction with water at 60°C for 24 hours |
| 6 | Japanese red pine, dried product of pine needles derived from adult trees | Extraction with 50% by mass aqueous solution of ethanol at 20°C for 30 minutes |
| 7 | Japanese red pine, dried product of pine needles derived from adult trees | Reflux with ethanol at 76°C for 1 hour |

[Experimental Example 3]

(Component analysis of pine needle extracts of Production Examples 1 to 7)

Analysis of D-pinitol, shikimic acid, and resin acid components contained in the pine needle extracts of Production Examples 1 to 7 was conducted by gas chromatography mass spectrometry (GC/MS) and HPLC. Specifically, D-pinitol and shikimic acid in the pine needle extracts were analyzed by HPLC, and the resin acids in the pine needle extracts were analyzed by GC/MS. In the analysis conducted by GC/MS, the sample was reacted with diazomethane to be methyl-esterified. Furthermore, the amounts of D-pinitol, shikimic acid, and resin acids were calculated as amounts calculated relative to pyrocatechol by creating a calibration curve using pyrocatechol.

The measuring apparatus and measurement conditions for GC/MS were as follows.

GC: Agilent 7890A (manufactured by Agilent Technologies, Inc.)
MS: Agilent 5977A (manufactured by Agilent Technologies, Inc.)
Autosampler: MPS robotic pro (manufactured by GERSTEL, Inc.)
Column: VF-5ms (0.25 mm × 0.25 μm × 30 m)
Carrier gas: He

The measuring apparatus and measurement conditions for HPLC were as follows.

Column: Inert Sustain Amide (3.0 × 250 mm i.d., 3 mm, manufactured by GL Sciences, Inc.)
Column oven temperature: 40°C
Sample dilution solvent: Methanol
Injection volume: 1 μL
Sample concentration: 50% by mass
Flow rate: 0.5 mL/min
UV detector: 210 nm
Mobile phase A: Acetonitrile
Mobile phase B: 100 mM aqueous solution of ammonium formate
Mobile phase composition conditions A:B = 80:20
Measurement time: 20 min

The following Tables 2 and 3 show the abundances of D-pinitol, shikimic acid, and resin acids in each pine needle extract. In the following Tables 2 and 3, the unit of the abundance of each component is ppm by mass. The amounts of D-pinitol and shikimic acid are values calculated relative to a case where the non-volatile content of the pine needle extract is set to 3% by mass. The method for measuring the non-volatile content of the pine needle extract was as follows.

B g of the pine needle extract was weighed out into a 20-mL screw tube (A g) whose mass had been measured, the pine needle extract was dried in a fair wind dryer at 80°C for 4 hours, and then the container was left to cool in a desiccator. The mass (C g) of the screw tube after cooling was measured, and the non-volatile content of the pine needle extract was calculated using the following expression.

$$\text{Non-volatile content (mass\%) of pine needle extract} = (C - A)/B \times 100$$

For example, when the non-volatile content of a pine needle extract is calculated to be 1.5% by mass by the above-described measurement method, and the pine needle extract contains 1,000 ppm of D-pinitol and 1,000 ppm of shikimic acid, the values calculated relative to the case where the non-volatile content is set to 3% by mass are calculated to be 2,000 ppm for D-pinitol and 2,000 ppm of shikimic acid.

[Table 2]

| Production Example | 1 | 2 | 3 | 4 | 5 | 6 |
|---|---|---|---|---|---|---|
| D-pinitol | 2503 | 3564 | 4444 | 2212 | 3259 | 9060 |
| Shikimic acid | 2342 | 6589 | 3400 | 2514 | 8060 | 2430 |

[Table 3]

| Production Example | 1 | 2 | 3 | 4 | 5 | 6 | 7 |
|---|---|---|---|---|---|---|---|
| Pimaric acid | 35 | 0 | 0 | 0 | 0 | 109 | 475 |
| Communic acid | 200 | 0 | 0 | 0 | 0 | 81 | 4436 |
| Isomer of pimaric acid or communic acid | 9 | 0 | 0 | 0 | 0 | 198 | 680 |
| Palustric acid | 0 | 0 | 0 | 0 | 0 | 268 | 624 |
| Dehydroabietic acid | 55 | 0 | 0 | 0 | 0 | 455 | 573 |
| Abietic acid | 6 | 0 | 0 | 0 | 0 | 83 | 115 |

As a result, it was shown that the pine needle extracts of Production Examples 1 to 7 contained 500 to 9,500 ppm of D-pinitol and 30 to 8,500 ppm of shikimic acid when the non-volatile content was set to 3% by mass. Furthermore, it was shown that the pine needle extracts of Production Examples 6 and 7 contained a large amount of resin acids.

[Experimental Example 4]

(Examination of cytotoxicity of pine needle extract of Production Example 1)

The cytotoxicity of the pine needle extract of Production Example 1 was examined. Regarding the cells, human primary hair papilla cells (HFDPC, purchased from PromoCell GmbH), which are mesenchymal cells isolated from the papilla of normal human scalp hair follicles, were used. HFDPCs were seeded on a collagen-coated 96-well plate at a concentration of $5 \times 10^4$ cells/well and incubated at 37°C in a humidified atmosphere of 5% $CO_2$. As a culture medium, a hair papilla cell growth culture medium to which growth factors (bovine fetal serum, insulin, transferrin, triiodothyronine, bovine pituitary extract, and a cyproterone solution) had been added was used.

After 24 hours, the culture medium for the cells was replaced with a culture medium to which a pine needle extract was added at a volume of 0 (control), 1/6,000, 1/5,000, 1/4,000, 1/3,000, 1/2,000, 1/1,000, 1/500, 1/250, or 1/100, and the cells were incubated for 48 hours.

Subsequently, the cell viability was measured using 3-(4,5-dimethyl-thiazol-2-yl)-2,5-diphenyltetrazolium bromide (MTT). Specifically, an MTT reagent (5 mg/mL) was added to the cells, the cells were incubated for 8 hours, subsequently 10% sodium dodecyl sulfate (SDS) was added thereto, and the cells were incubated overnight. Thereafter, the absorbance at 570 nm was measured using a microplate reader, and the cell viability was quantified as a proportion (%) of the absorbance with respect to the absorbance of untreated cells.

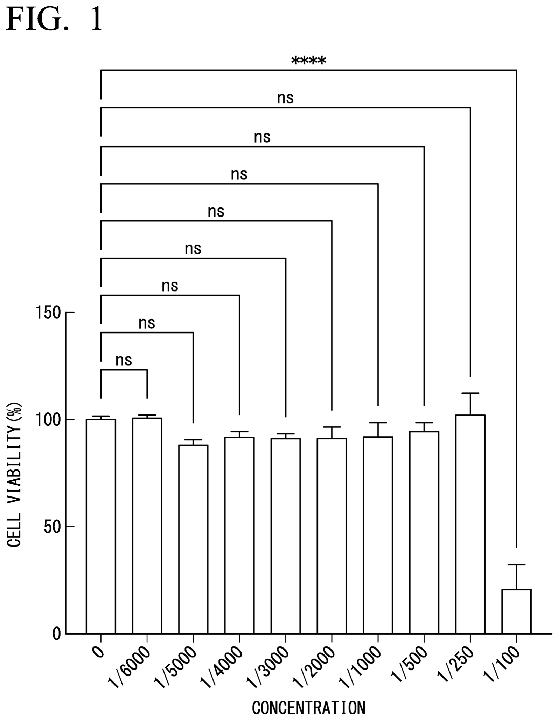

FIG. 1 is a graph showing the measurement results for the cell viability. In FIG. 1, "ns" indicates that there is no significant difference, and "****" indicates that there is a significant difference with P < 0.0001. As a result, it was found that even when the pine needle extract was added to the culture medium at a volume of 1/6,000 to 1/250, cytotoxicity was not exhibited. When the pine needle extract was added to the culture medium at a volume of 1/100, the cell viability decreased to about 30%.

[Experimental Example 5]

(Examination of cytotoxicity of pine needle extracts of Production Examples 3, 4, 6, and 7)
The cytotoxicity of the pine needle extracts of Production Examples 3, 4, 6, and 7 was examined by the same method as that used in Experimental Example 4. The pine needle extract of Production Example 6 had slightly higher cytotoxicity, as was the case with the pine needle extract of Production Example 1. Furthermore, the pine needle extract of Production Example 7 had high cytotoxicity, and the influence on hair growth-related genes as will be described later could not be examined.

The results of the examination are shown in the following Table 4. In the following Table 4, "Cytotoxicity" indicates the evaluation results for cytotoxicity. In the following Table 4, the evaluation criteria for cytotoxicity were as follows.

(Evaluation criteria for cytotoxicity)

-: No cytotoxicity was observed.
±: The cell viability was reduced by 40% or more at a volume of 1/100.
+: The cell viability was reduced by 10% or more at a volume of 1/10,000.
++: The cell viability was reduced by 40% or more at a volume of 1/10,000.

[Table 4]

| Production Example | Cytotoxicity |
|---|---|
| Production Example 1 | ± |
| Production Example 3 | - |
| Production Example 4 | - |
| Production Example 6 | ± |
| Production Example 7 | ++ |
| D-pinitol | - |
| Shikimic acid | - |

[Experimental Example 6]

(Examination of effect of pine needle extract of Production Example 1 on hair growth-related genes)

The pine needle extract of Production Example 1 was added to the culture medium for HFDPCs, and the expression of hair growth markers was measured by quantitative real-time PCR. As the hair growth markers, CTNNB1 gene (β-catenin), ALPL gene (alkaline phosphatase), and FGF1 gene (fiblobrast growth factor 1) were examined. It is known that β-catenin induces anagen of the hair cycle and promotes the proliferation of keratinocytes. ALPL is a representative hair growth marker in the hair papilla cells. FGF1 is a marker for morphogenesis of hair follicles.

HFDPCs were seeded in a 6-well plate at a concentration of $5 \times 10^4$ cells/well and incubated at 37°C for 24 hours. Subsequently, the culture medium was removed and replaced with a culture medium containing a pine needle extract at a volume of 1/4,000 and 1/2,000. Furthermore, for a comparison, cells treated with a culture medium to which minoxidil (0.1 μM), an existing therapeutic agent for hair loss, was added, were also prepared.

Subsequently, after 48 hours, the cells were washed with cold PBS, and total RNA was extracted using an ISOGEN kit (Nippon Gene Co., Ltd.). Subsequently, the total RNA was quantified using a NanoDrop 2000 spectrophotometer (Thermo Fisher Scientific, Inc.), and quantitative real-time PCR analysis was performed.

First, using a SuperScript III reverse transcription kit (Thermo Fisher Scientific, Inc.), a cDNA was synthesized from the extracted total RNA by a cycling protocol of 95°C for 10 minutes, 40 cycles of 95°C for 15 seconds, and 60°C for 1 minute.

Subsequently, real-time PCR was carried out using a 7500 Fast Real-Time PCR system (Software 1.3.1, Thermo Fisher Scientific, Inc.) and TaqMan probes specific for CTNNB1, ALPL, and FGF1. GAPDH was used as an endogenous control, and the $2^{-\triangle\triangle Ct}$ method was applied, to calculate the relative amount of mRNA in comparison with GAPDH.

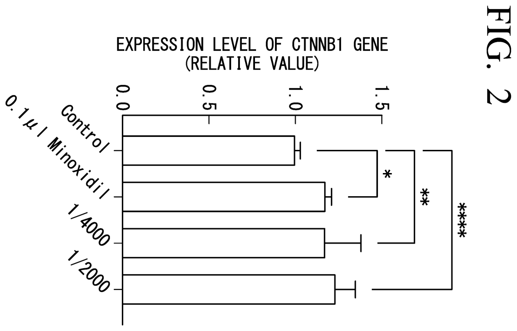

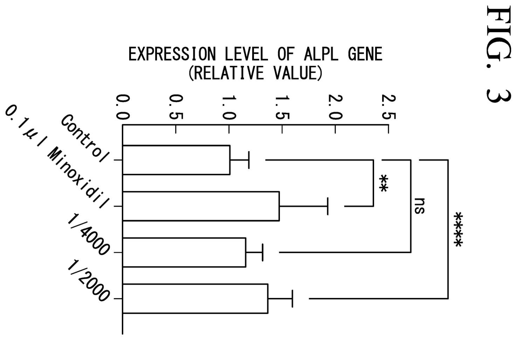

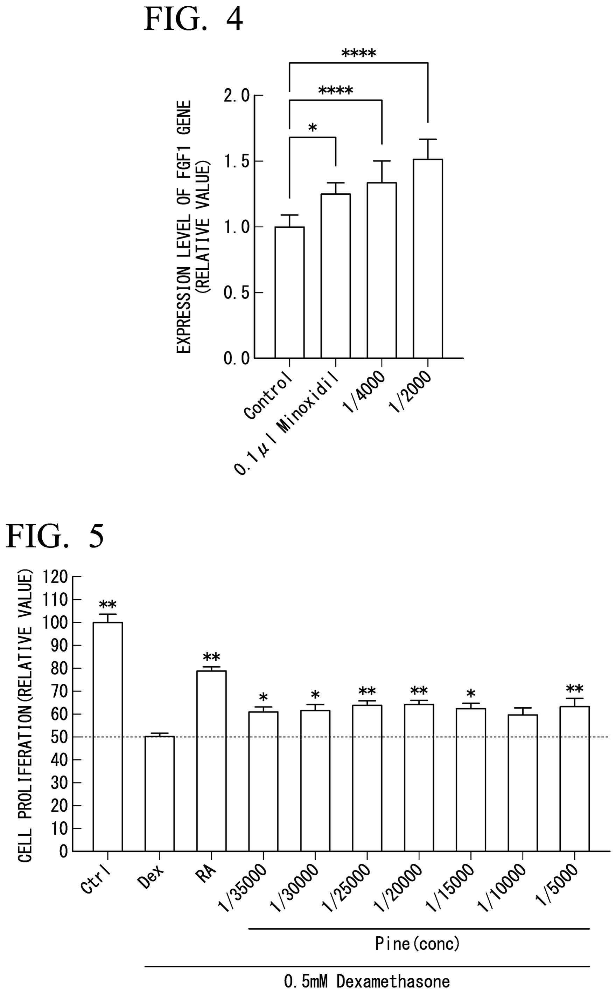

FIG. 2 is a graph showing the results of quantitative real-time PCR of CTNNB1 gene. FIG. 3 is a graph showing the results of quantitative real-time PCR of ALPL gene. FIG. 4 is a graph showing the results of quantitative real-time PCR of FGF1 gene. In FIGS. 2 to 4, the values in the graphs indicate the mean value ± standard deviation (n = 3). Furthermore, "ns" indicates that there is no significant difference, "*" indicates that there is a significant difference with P < 0.05, "**" indicates that there is a significant difference with P < 0.01, and "****" indicates that there is a significant difference with P < 0.0001.

As a result, it was found that the expression levels of CTNNB1 gene and FGF1 gene in HFDPCs were significantly increased by a culture medium containing the pine needle extract at a volume of 1/4,000 or 1/2,000. Furthermore, it was found that the expression level of ALPL gene in HFDPCs was significantly increased by a culture medium containing a pine needle extract at a volume of 1/2,000. Furthermore, the effects of increasing the expression level of FGF1 gene were superior to the effects of the treatment with 0.1 $\mu$M minoxidil, which was a pharmaceutical drug for hair growth used as a positive control. This result indicates that the pine needle extract has hair growth promoting effects.

[Experimental Example 7]

(Examination of effect of pine needle extracts of Production Examples 3 to 6 on hair growth-related genes)

The effect of the pine needle extracts of Production Examples 3 to 6 on the hair growth-related genes was examined by the same method as that used in Experimental Example 6. Furthermore, the same examination was carried out for D-pinitol and shikimic acid. The pine needle extracts of Production Examples 3 to 6 were each added to the culture medium for cells at a volume of 1/6,000 or a volume of 1/10,000. The pine needle extracts of Production Examples 1 and 6 were each added to the culture medium for cells at a concentration at which cytotoxicity was not observed. D-pinitol was added to the culture medium for cells at 1 $\mu$M and 10 $\mu$M. Shikimic acid was added to the culture medium for cells at 1 $\mu$M and 10 $\mu$M.

The results of the examination are shown in the following Table 5. In the following Table 5, "CTNNB1", "ALPL", and "FGF1" indicate the evaluation results for the expression enhancing effects of each hair growth-related gene. In the following Table 5, the evaluation criteria for the effect on the hair growth-related genes were as follows.

(Evaluation criteria for effect on hair growth-related genes)

-: Expression was suppressed.
$\pm$: No significant difference was observed in the expression enhancing effects at at least some of the concentrations.
+: A significant difference with p < 0.05 or more was observed in the expression enhancing effects at all concentrations.
++: A significant difference with p < 0.01 or more was observed in the expression enhancing effects at all concentrations.

[Table 5]

|  | ALPL | CTNNB1 | FGF1 |
|---|---|---|---|
| Production Example 1 | $\pm$ | + | ++ |
| Production Example 3 | + | $\pm$ | - |
| Production Example 4 | ++ | ++ | + |
| Production Example 6 | ++ | ++ | ++ |
| Production Example 7 | ++ | ++ | ++ |
| D-pinitol | - | ++ | ++ |
| Shikimic acid | $\pm$ | + | ++ |

As a result, D-pinitol and shikimic acid exhibited expression enhancing effects for CTNNB1 and FGF1 but did not exhibit expression enhancing effects for ALPL gene. On the other hand, the pine needle extracts of Production Examples 3 to 6 exhibited expression enhancing effects for ALPL. Furthermore, the pine needle extracts of Production Examples 4 to 6 exhibited expression enhancing effects for all of CTNNB1, ALPL, and FGF1.

[Experimental Example 8]

(Examination of neuroprotective action of pine needle extract of Production Example 1)

The neuroprotective action (antidepressant action and anti-stress action) of the pine needle extract was examined using an evaluation system for a depression model in vitro. More specifically, SH-SY5Y cells, which are a human neuroblastoma cell line, were treated with dexamethasone as a neurotoxin in the presence of a pine needle extract, and it was examined whether the dexamethasone-induced neuronal cell death was suppressed.

SH-SY5Y cells were seeded in a 96-well plate at a concentration of $2.0 \times 10^4$ cells/well and cultured at 37°C in the presence of 5% $CO_2$ for 24 hours. As a culture medium, a culture medium obtained by adding 15% fetal bovine serum (FBS), 1% penicillin/streptomycin, and 1% non-essential amino acids to a mixture of DMEM and Ham's F-12 nutrient mixture at a ratio of 1:1 (v:v), was used.

After 24 hours, the culture medium was replaced with a culture medium to which a pine needle extract was added at a volume of 0 (control), 1/35,000, 1/30,000, 1/25,000, 1/20,000, 1/15,000, 1/10,000, or 1/5,000, and the cells were incubated for 1 hour. Furthermore, for a comparison, cells treated with a culture medium to which rosmarinic acid (10 μM) known to have a neuroprotective action was added, were also prepared. After 1 hour, 0.5 mM dexamethasone was added to each well, and the cells were further incubated for 48 hours.

Subsequently, an MTT reagent (5 mg/mL) was added to the cells, the cells were incubated for 24 hours, subsequently 10% sodium dodecyl sulfate (SDS) was added thereto, and the cells were incubated for 24 hours. Thereafter, the absorbance at 570 nm was measured using a microplate reader, and the cell proliferation was quantified as a proportion (%) of the absorbance with respect to the absorbance of untreated cells.

FIG. 5 is a graph showing the results of evaluating cell proliferation. In FIG. 5, a value in the graph indicates the mean value $\pm$ standard deviation (n = 6). Furthermore, "Ctrl" indicates the results of the control in which neither dexamethasone nor the pine needle extract was added, "Dex" indicates the results obtained by adding only dexamethasone, "RA" indicates the results obtained by adding rosmarinic acid, and "Pine" indicates the results obtained by adding the pine needle extract. Furthermore, "*" indicates that there is a significant difference with $P < 0.05$ as compared with cells to which only dexamethasone was added, and "**" indicates that there is a significant difference with $P < 0.01$ as compared with cells to which only dexamethasone was added.

As a result, higher cell proliferation was observed in the SH-SY5Y cells to which the pine needle extract had been added to the culture medium, as compared with the cell proliferation in the SH-SY5Y cells to which only dexamethasone had been added. These results indicate that the pine needle extract has a significant neuroprotective action against dexamethasone. These results indicate that the pine needle extract is useful as an antidepressant and an anti-stress agent.

[Experimental Example 9]

(Examination of effect of pine needle extract exerted on depressive behavior)

As the pine needle extract, the pine needle extract of Production Example 2 was used, and the effect of the pine needle extract exerted on the in vivo depressive behavior was examined by a tail suspension test using mice. The tail suspension test (TST) is a test for measuring the time during which a mouse suspended upside down struggles, and is used as an indicator of depressive-like behavior. The suspended mouse moves around in an attempt to escape; however, the time with no movement (immobility time) gradually increases. It is known that the immobility time in the tail suspension test is reduced in mice to which an antidepressant is administered. It is interpreted that an increase in the immobility time indicates an increase in depressive-like behavior, and a decrease in the immobility time indicates a decrease in depressive-like behavior.

Mice of each group as shown in the following Table 6 were prepared. In the following Table 6, "LPS" indicates lipopolysaccharide. Furthermore, bupropion administered to a positive control group is an antidepressant. The mice in each group were kept for 7 days to be acclimatized, and then a pine needle extract was orally administered daily for 7 days. Thereafter, neuroinflammation was induced in the brain by a single intraperitoneal administration of LPS, and depression was developed. A tail suspension test was performed the day after the administration of LPS. In the tail suspension test, a SMART 3.0 video image behavior analysis device (Panlab S.L.U.) was used. The brain was removed the day after the tail suspension test, and the hippocampus was further collected and used in the experiment that will be described later.

[Table 6]

| | Mouse | Oral administration | Intraperitoneal administration | n |
|---|---|---|---|---|
| Control group | ICR mouse, male, 8 weeks old | Physiological saline | Physiological saline | 6 |
| LPS-administered group | ICR mouse, male, 8 weeks old | Physiological saline | 0.85 mg/kg LPS/physiological saline | 7 |

(continued)

|  | Mouse | Oral administration | Intraperitoneal administration | n |
|---|---|---|---|---|
| Positive control group | ICR mouse, male, 8 weeks old | 20 mg/kg bupropion/physiological saline | 0.85 mg/kg LPS/physiological saline | 6 |
| Low-dose pine needle extract-administered group | ICR mouse, male, 8 weeks old | 5 mg/kg pine needle extract/physiological saline | 0.85 mg/kg LPS/physiological saline | 6 |
| High-dose pine needle extract-administered group | ICR mouse, male, 8 weeks old | 50 mg/kg pine needle extract/physiological saline | 0.85 mg/kg LPS/physiological saline | 6 |

The following Table 7 shows the changes in body weight and the total amount of food intake of the mice in each group during the oral administration period of the pine needle extract. As a result, oral administration of the pine needle extract for 7 days did not affect the body weight and the total amount of food intake. From this result, it was considered that the pine needle extract does not exhibit serious toxicity.

[Table 7]

|  | Initial body weight (g) | Final body weight (g) | Increase in body weight (g / 7 days) | Total amount of food intake (g / 7 days) |
|---|---|---|---|---|
| Control group | 34.0 ± 0.3 | 36.5 ± 0.9 | 2.5 ± 0.4 | 24.1 ± 1.2 |
| LPS-administered group | 32.4 ± 0.4 | 35.5 ± 0.5 | 3.1 ± 0.3 | 24.6 ± 1.4 |
| Positive control group | 32.7 ± 0.8 | 35.6 ± 0.7 | 2.9 ± 0.4 | 25.0 ± 1.2 |
| Low-dose pine needle extract-administered group | 32.5 ± 0.5 | 35.1 ± 0.5 | 2.6 ± 0.1 | 23.0 + 0.3 |
| High-dose pine needle extract-administered group | 32.4 ± 1.0 | 35.3 ± 0.8 | 2.9 ± 0.4 | 23.3 ± 0.9 |

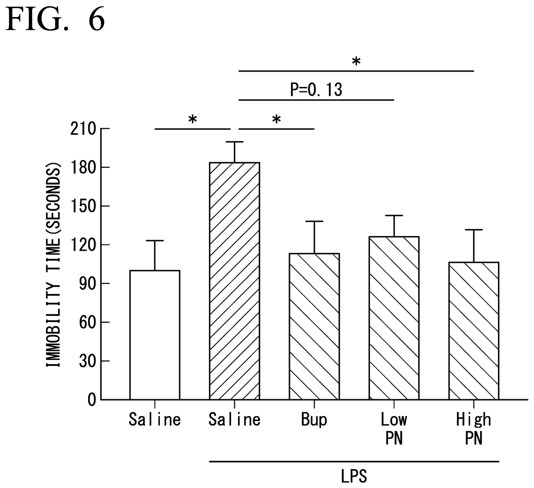

FIG. 6 is a graph showing the results of the tail suspension test. In FIG. 6, "Saline" indicates physiological saline, "Bup" indicates bupropion, "LowPN" indicates the results of a low-dose pine needle extract-administered group, and "HighPN" indicates the results of a high-dose pine needle extract-administered group. Furthermore, "*" indicates that there is a significant difference with $p < 0.05$.

As a result, it was found that the oral administration of a high-dose pine needle extract (50 mg/kg) significantly suppressed the prolongation of the immobility time caused by LPS-induced neuroinflammation. On the other hand, no significant effects were observed in the oral administration of a low-dose pine needle extract (5 mg/kg). These results indicate that the pine needle extract has a depressive behavior suppressing effect.

[Experimental Example 10]

(Analysis of protein expression in mouse hippocampus)

The hippocampi of the mice in each group collected in Experimental Example 9 were subjected to ELISA, and mature BDNF, dopamine, noradrenaline, serotonin, and acetylcholine were quantified.

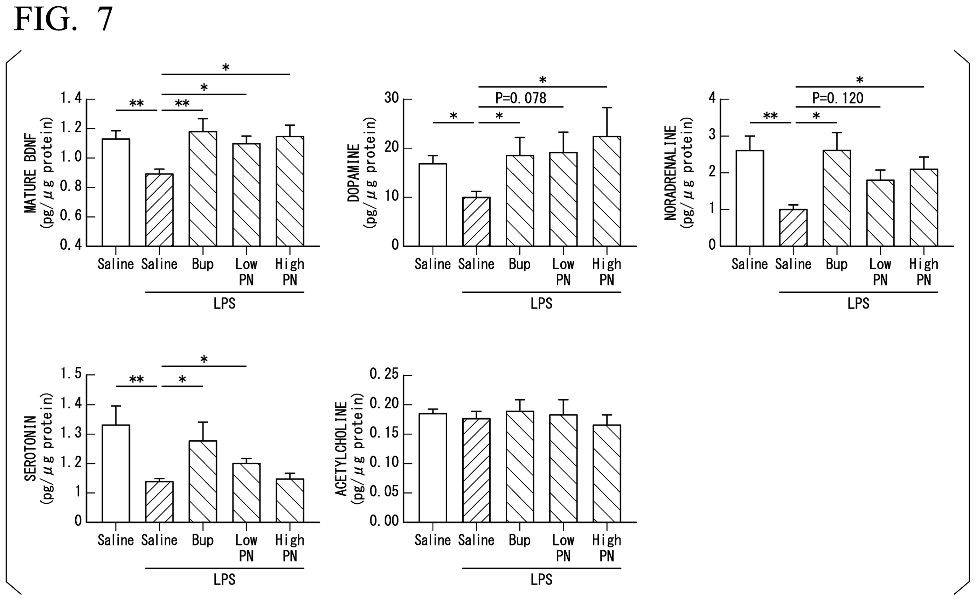

FIG. 7 is a graph showing the quantification results. In FIG. 7, "Saline" indicates physiological saline, "Bup" indicates bupropion, "LowPN" indicates the results of a low-dose pine needle extract-administered group, and "HighPN" indicates the results of a high-dose pine needle extract-administered group. Furthermore, "*" indicates that there is a significant difference with $p < 0.05$, and "**" indicates that there is a significant difference with $p < 0.01$.

As a result, it was found that oral administration of the pine needle extract significantly suppressed a decrease in the expression of mature BDNF caused by LPS-induced neuroinflammation. Furthermore, it was found that a decrease in the expression levels of dopamine and noradrenaline caused by LPS-induced neuroinflammation was significantly suppressed in the high-dose pine needle extract-administered group. Furthermore, it was found that a decrease in the expression level of serotonin caused by LPS-induced neuroinflammation was significantly suppressed in the low-dose pine needle extract-administered group. On the other hand, it was shown that oral administration of the pine needle extract did not affect the expression of acetylcholine.

[Experimental Example 11]

(Comprehensive analysis of gene expression in mouse hippocampus)

The hippocampi of the mice in each group collected in Experimental Example 9 were subjected to DNA microarray analysis, and the gene expression in the high-dose pine needle extract-administered group and the LPS-administered group was comprehensively analyzed.

The number of genes whose expression ratio (Fold Change) exceeding 1.5 between the high-dose pine needle extract-administered group and the LPS-administered group was such that the number of genes whose expression increased was 330, and the number of genes whose expression decreased was 196. The expression ratio was calculated using the following formula.

Expression ratio = Expression level in high-dose pine needle extract-administered group/expression level in LPS-administered group

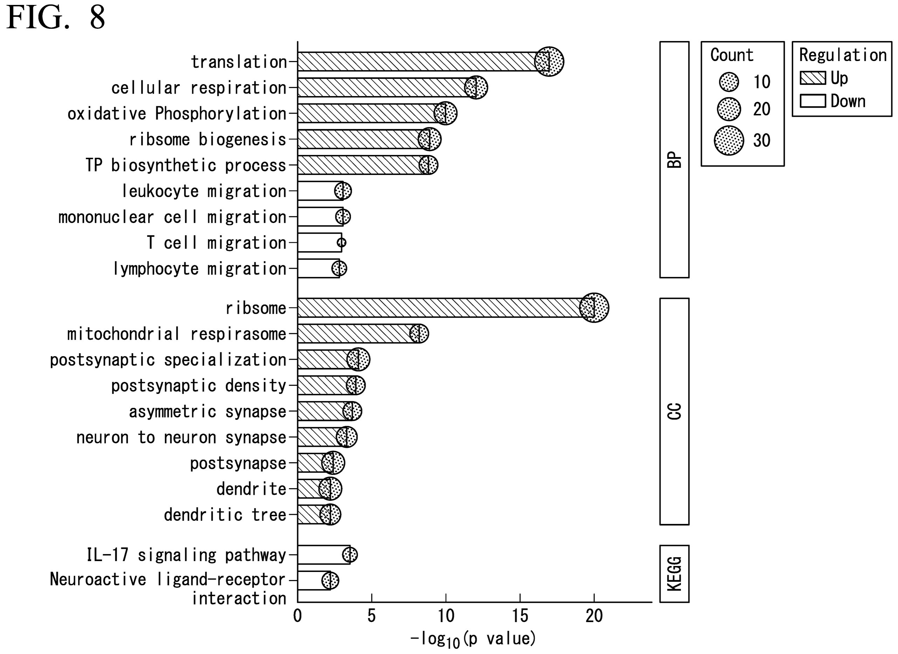

FIG. 8 is a diagram showing the results of performing Gene Ontology (GO) analysis on the group of genes whose expression was altered in the high-dose pine needle extract-administered group. In FIG. 8, "BP" indicates biological process, "CC" indicates cell constituent, and "KEGG" indicates Kyoto Encyclopedia of Genes and Genomes (KEGG), which is a database that associates enzyme reactions, signal transduction, and the like centered on metabolic pathways, with genes and compounds. As a result, it was shown that the expression of a group of genes involved in metabolism and nerves was increased, and the expression of a group of genes involved in inflammation was decreased. From this result, it was suggested that there is a possibility that functions of neurogenesis, suppressing neuroinflammation, and improving neurometabolism may be exhibited by administration of a pine needle extract.

INDUSTRIAL APPLICABILITY

According to the present invention, a vitality improving agent derived from a natural product can be provided.

**Claims**

1. A vitality improving agent comprising a pine needle extract as an active ingredient.

2. The vitality improving agent according to Claim 1,
   wherein the pine needle extract is an extract obtained using water or a mixed liquid of water and an alcohol as an extraction solvent.

3. The vitality improving agent according to Claim 1 or 2,
   wherein the pine needle extract contains 500 to 9,500 ppm by mass of D-pinitol and 30 to 8,500 ppm by mass of shikimic acid.

4. The vitality improving agent according to Claim 1 or 2,
   wherein the pine needle extract contains substantially no resin acids.

5. A composition for improving vitality, comprising:

   the vitality improving agent according to Claim 1 or 2; and
   a pharmaceutically acceptable carrier.

6. A food composition for improving vitality, comprising the vitality improving agent according to Claim 1 or 2.

FIG. 1

FIG. 2

FIG. 3

# FIG. 4

# FIG. 5

FIG. 6

FIG. 7

EP 4 702 982 A1

FIG. 8

# INTERNATIONAL SEARCH REPORT

| International application No. |
| --- |
| **PCT/JP2024/016081** |

**A. CLASSIFICATION OF SUBJECT MATTER**

*A61K 36/15*(2006.01)i; *A23L 33/105*(2016.01)i; *A61K 8/9767*(2017.01)i; *A61P 17/14*(2006.01)i; *A61P 25/24*(2006.01)i; *A61Q 7/00*(2006.01)i

FI: A61K36/15; A23L33/105; A61K8/9767; A61P17/14; A61P25/24; A61Q7/00

According to International Patent Classification (IPC) or to both national classification and IPC

**B. FIELDS SEARCHED**

Minimum documentation searched (classification system followed by classification symbols)

A61K36/15; A23L33/105; A61K8/9767; A61P17/14; A61P25/24; A61Q7/00

Documentation searched other than minimum documentation to the extent that such documents are included in the fields searched

Published examined utility model applications of Japan 1922-1996
Published unexamined utility model applications of Japan 1971-2024
Registered utility model specifications of Japan 1996-2024
Published registered utility model applications of Japan 1994-2024

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C. DOCUMENTS CONSIDERED TO BE RELEVANT**

| Category* | Citation of document, with indication, where appropriate, of the relevant passages | Relevant to claim No. |
| --- | --- | --- |
| X | KURIBARA, H et al. An antidepressant effect of Sho-ju-sen, a Japanese herbal medicine, assessed by learned helplessness model in mice. Phytotherapy research. 2004, vol. 18, no. 2, pp. 173-176 abstract, table 3 | 1-6 |
| X | 栗原　久ほか, 生薬製剤「松寿仙」の意欲改善効果, 新薬と臨床, 2002, vol. 51, no. 1, pp. 22-29, (KURIHARA, Hisashi et al. A Tonic Effect of Sho-ju-sen, a Japanese Herbal Medicine. Journal of New Remedies & Clinics.) summary, fig. 1 | 1-6 |
| X | LEE, JS et al. Pine needle extract prevents hippocampal memory impairment in acute restraint stress mouse model. J. Ethnopharmacol. 2017, vol. 207, pp. 226-236 abstract, 2.1, fig. 2 | 1-6 |
| X | JP 2011-225506 A (HIGAMI, Yoshiaki) 10 November 2011 (2011-11-10) examples | 1-6 |

[✓] Further documents are listed in the continuation of Box C.     [✓] See patent family annex.

* Special categories of cited documents:
"A" document defining the general state of the art which is not considered to be of particular relevance
"D" document cited by the applicant in the international application
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
| --- | --- |
| **26 June 2024** | **09 July 2024** |

| Name and mailing address of the ISA/JP | Authorized officer |
| --- | --- |
| **Japan Patent Office (ISA/JP)** **3-4-3 Kasumigaseki, Chiyoda-ku, Tokyo 100-8915 Japan** | |
| | Telephone No. |

Form PCT/ISA/210 (second sheet) (July 2022)

**INTERNATIONAL SEARCH REPORT**

| International application No. |
|---|
| **PCT/JP2024/016081** |

**C.    DOCUMENTS CONSIDERED TO BE RELEVANT**

| Category* | Citation of document, with indication, where appropriate, of the relevant passages | Relevant to claim No. |
|---|---|---|
| X | JP 2010-514756 A (KIM, Ji-Hwal) 06 May 2010 (2010-05-06)<br>claims, example 2 | 1-5 |
| A | claims, example 2 | 6 |
| X | JP 5-339133 A (KABUSHIKI KAISHA HAYASHIBARA SEIBUTSU KAGAKU KENKYUJO) 21 December 1993 (1993-12-21)<br>examples | 1-5 |
| A | examples | 6 |
| A | JP 6-9419 A (KABUSHIKI KAISHA HAYASHIBARA SEIBUTSU KAGAKU KENKYUJO) 18 January 1994 (1994-01-18)<br>examples | 1-6 |
| A | JP 54-59311 A (MIDORIKAWA, Yoshinobu) 12 May 1979 (1979-05-12)<br>examples | 1-6 |
| A | JANG, JY et al. Effect of pine needle water extract on cadmium-induced oxidative stress in rats. J. Korean Soc. Food Sci. Nutr. 2007, vol. 36, no. 4 , pp. 411-418<br>abstract | 1-6 |
| A | JP 2003-155219 A (INOUE, Tadao) 27 May 2003 (2003-05-27)<br>claims, examples | 1-6 |

Form PCT/ISA/210 (second sheet) (July 2022)

## INTERNATIONAL SEARCH REPORT
### Information on patent family members

| Patent document cited in search report | | | Publication date (day/month/year) | Patent family member(s) | | | Publication date (day/month/year) |
|---|---|---|---|---|---|---|---|
| JP | 2011-225506 | A | 10 November 2011 | (Family: none) | | | |
| JP | 2010-514756 | A | 06 May 2010 | US 2010/0104646 claims, example 2 WO 2008/082123 claims, example 2 KR 10-0757237 claims, example 2 CN 101646423 claims, example 2 | A1 A1 B1 A | | |
| JP | 5-339133 | A | 21 December 1993 | US 5494667 examples EP 573141 examples KR 10-1994-0005260 examples CN 1082398 examples | A A2 A A | | |
| JP | 6-9419 | A | 18 January 1994 | US 5466453 examples EP 565313 examples KR 10-1993-0021110 examples CN 1081844 examples | A A2 A A | | |
| JP | 54-59311 | A | 12 May 1979 | (Family: none) | | | |
| JP | 2003-155219 | A | 27 May 2003 | (Family: none) | | | |

Form PCT/ISA/210 (patent family annex) (July 2022)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2023075024 A
- US 4139619 A
- US 20020042425 A
- US 2004121010 A